# NEUE EUROPÄISCHE PATENTSCHRIFT

(11) **EP 1 132 371 B2**
(45) Veröffentlichungstag und Bekanntmachung des Hinweises auf die Entscheidung über den Einspruch: **20.08.2008**
(45) Hinweis auf die Patenterteilung: 09.07.2003
(21) Anmeldenummer: 01104959.0
(22) Anmeldetag: 01.03.2001
(51) Int. Cl.: C07C 213/10

(54) **Verfahren zur Herstellung von Triäthanolamin oder N-(2-Aminoäthyl)-äthanolamin mit verbesserter Farbqualität**
Process for the preparation of triethanolamine or N-(2-aminoethyl)-ethanolamine with improved colour quality
Procédé de préparation de la triéthanolamine ou N-(2-aminoéthyle)-éthanolamine avec une qualité de couleur améliorée

(30) Priorität: 11.03.2000 DE 10011942
(43) Veröffentlichungstag der Anmeldung: 12.09.2001
(73) Patentinhaber: BASF SE, 67056 Ludwigshafen (DE)
(72) Erfinder: Ruider, Günther, Dr., 67157 Wachenheim (DE); Ross, Karl-Heinz, Dr., 67269 Grünstadt (DE); Melder, Johann-Peter, Dr., 67459 Böhl-Iggelheim (DE); Schulz, Gerhard, Dr., 67069 Ludwigshafen (DE); Gutschoven, Frank, 2018 Anwerpen (BE); Buskens, Philip, Dr., 2320 Hoogstraten (BE)
(74) Vertreter: Isenbruck, Günter

(56) Entgegenhaltungen:
- EP-A- 0 004 015
- WO-A-00/32553
- DATABASE WPI Section Ch, Week 199248 Derwent Publications Ltd., London, GB; Class E16, AN 1992-393250 XP002167743 & JP 04 290850 A (MITSUI TOATSU CHEM INC), 15. Oktober 1992 (1992-10-15)
- DIN 6174 (CIELAB-Formel)
- H.W. SCHEELINE: 'Ethylene Glycols, Glycol Ethers and Ethanolamines' SRI INTERNATIONAL, REPORT NR 70 August 1971, MENLO PARK, CALIFORNIA, US, Seiten 145 - 150

## Beschreibung

Die vorliegende Erfindung betrifft ein Verfahren zur Herstellung von Triethanolamin oder N-(2-Aminoethyl)-ethanolamin mit verbesserter Farbqualität.

Wichtige Einsatzgebiete von Alkanolaminen, wie z. B. Triethanolamin (TEA), oder deren Folgeprodukten sind beispielsweise Seifen, Waschmittel und Shampoos in der kosmetischen Industrie oder auch Dispergiermittel und Emulgiermittel.

Für diese und andere Einsatzgebiete sind wasserklare, farblose Alkanolamine mit einer möglichst geringen Verfärbung, z. B. gemessen als APHA- oder Gardner-Farbzahl, die diese Eigenschaften auch über längere Lagerzeiten (von z. B. 6, 12 oder mehr Monaten) beibehalten, erwünscht.

Ein bekanntes Problem ist, dass ein nach einer fraktionierenden Destillation eines Alkanolamin-Rohprodukts, das z. B. durch Umsetzung von Ammoniak mit Ethylenoxid oder Propylenoxid gewonnen wurde, erhaltenes reines Alkanolamin eine gelbliche bis bräunliche Verfärbung aufweist (Farbzahl z. B. ca. 10 bis 500 APHA nach DIN ISO 6271(= Hazen)). Diese Verfärbung tritt besonders in Prozessen auf, in denen hohe Temperaturen durchlaufen werden.

Bei einer Lagerung des Alkanolamins, auch im geschlossenen Gebinde und unter Lichtausschluss, wird diese Verfärbung noch weiter verstärkt. (Siehe z. B.: T.I. MacMillan, Ethylene Oxide Derivatives, Report No. 193, Kapitel 6, Seiten 6-5 und 6-9 bis 6-13, 1991, SRI International, Menlo Park, California 94025;
G.G. Smirnova et al., J. of Applied Chemistry of the USSR 61, S. 1508-9 (1988), und Chemical & Engineering News 1996, Sept. 16, Seite 42, mittlere Spalte).

In der Literatur sind verschiedene Verfahren zur Herstellung von Alkanolaminen mit verbesserter Farbqualität beschrieben.

EP-A-36 152 und EP-A-4015 erläutern den Einfluss der in Verfahren zur Herstellung von Alkanolaminen eingesetzten Werkstoffe auf die farbliche Qualität der Verfahrensprodukte und empfehlen nickelfreie bzw. nickelarme Stähle.

US-A-3 207 790 beschreibt ein Verfahren zur Verbesserung der Farbqualität von Alkanolaminen durch Zugabe eines Borhydrids eines Alkalimetalls in das Alkanolamin.

Die Anwesenheit eines Hilfsstoffes (Stabilisators) zur Verbesserung der Farbqualität von Alkanolaminen ist jedoch in vielen wichtigen Anwendungsbereichen unerwünscht.

Die ältere deutsche Anmeldung Nr. 19942300.8 vom 04.09.99 betrifft ein Verfahren zur Herstellung von Alkanolaminen mit verbesserter Farbqualität durch Behandlung des Alkanolamins mit Wasserstoff in Gegenwart eines Hydrierkatalysators bei erhöhter Temperatur.

JP-A-04 29 0850 (Derwent Abstract Nr. 92-393250/48; Chem. Abstr. 118: 101513e) beschreibt die Entfärbung von Triethylentetramin durch Erhitzen in Gegenwart von phosphoriger Säure und Wasser.

JP-A-49 07 6804 (Derwent Abstract Nr. 76608V 44; Chem. Abstr. 82:3766h) betrifft die Reinigung von Ethylenaminen wie Triethylentetramin oder Pentaethylenhexaamin durch Destillation in Gegenwart von Estern der phosphorigen Säure.

EP-A-4015 beschreibt, dass Mono-, Di- und Triethanolamin mit geringerer Verfärbung durch Zusatz von phosphoriger oder unterphosphoriger Säure oder deren Derivate vor oder während oder direkt nach der stufenweisen Umsetzung von Ethylenoxid mit Ammoniak und anschließende Isolierung durch Destillation erhalten werden. Auf Seite 2, Zeilen 14 bis 18, wird gelehrt, dass es nicht gelingt, bereits mehr oder weniger verfärbte Ethanolamine durch eine in Gegenwart von phosphoriger Säure durchgeführte Destillation in befriedigendem Maße zu entfärben. EP-A-4015 lehrt, dass es vielmehr erforderlich ist, dass die phosphorige oder unterphosphorige Säure während der Ethanolaminherstellung zugegen ist oder zumindest direkt nach der Umsetzung in das rohe Reaktionsgemisch, enthaltend die Ethanolamine, Wasser und Ammoniak, zugegeben wird (vergl. die Beispiele und den Patentanspruch).

Die ältere deutsche Anmeldung Nr. 19855383.8 vom 01.12.98 betrifft ein Verfahren zur Reinigung von TEA, hergestellt durch Umsetzung von wässrigem Ammoniak mit Ethylenoxid in flüssiger Phase unter Druck und bei erhöhter Temperatur, indem man vom Umsetzungsprodukt überschüssiges Ammoniak, Wasser und Monoethanolamin abtrennt, das so erhaltene Rohprodukt mit Ethylenoxid bei Temperaturen von 110 bis 180°C umsetzt und anschließend in Gegenwart von phosphoriger oder unterphosphoriger Säure oder deren Verbindungen rektifiziert.

Der vorliegenden Erfindung lag die Aufgabe zugrunde, ein wirtschaftliches, selektives, effizientes und technisch unaufwendiges Verfahren zur Herstellung von Triethanolamin oder N-(2-Aminoethyl)-ethanolamin mit verbesserter Farbqualität aufzufinden. Das Verfahren soll es unter Überwindung der Nachteile des Stands der Technik ermöglichen, die Verfärbung von Triethanolamin oder Aminoethylethanolamin, z. B. gemessen als APHA-Farbzahl, zu vermindern und die Farbstabilität zu verbessern (unerwünschte Zunahme der Farbzahl über die Lagerzeit).

Demgemäß wurde ein Verfahren zur Herstellung von Triethanolamin oder N-(2-Aminoethyl)-ethanolamin mit verbesserter Farbqualität gefunden, welches dadurch gekennzeichnet ist, dass man das Alkanolamin, dessen Reinheit größer 70 Gew.-% beträgt, mit einer wirksamen Menge von phosphoriger oder unterphosphoriger Säure oder deren Verbindungen zunächst bei Temperaturen von 150 bis 220°C über einen Zeitraum von 30 Min. bis 2 Stunden behandelt (Schritt a) und anschließend in Gegenwart einer wirksamen Menge einer dieser Phosphorverbindungen-destilliert (Schritt b), wobei man die Behandlung des Alkanolamins in Schritt a und die Destillation des Alkanolamins (Schritt b) jeweils in Gegenwart von 0,02 bis 0,5 Gew.-% der Phosphorverbindung, bezogen auf die eingesetzte Menge an Alkanolamin, durchführt.

Das im erfindungsgemäßen Verfahren eingesetzte Ethanolamin kann nach bekannten Verfahren, z. B. durch Umsetzung von Ammoniak oder eines primären Amins mit Ethylenoxid (z. B. gemäß EP-A-673 920) oder Aminierung von entsprechenden primären Alkoholen erhalten werden.

N-(2-Aminoethyl)-ethanolamin (AEEA) kann durch Umsetzung von Monoethanolamin oder Ammoniak mit Ethylenoxid in Gegenwart von Wasserstoff und einem Hydrier-, Dehydrier- oder Aminierungskatalysator erhalten werden.

Die Reinheit der im erfindungsgemäßen Verfahren eingesetzten Ethanolamine, beträgt größer 70 Gew.-%, insbesondere größer 80 Gew.-%. Neben destillierten oder undestillierten rohen Alkanolaminen, die auch direkt in roher Form einer Anlage zur Herstellung des Alkanolamins aus den entsprechenden Vorstufen entnommen werden können, können auch destillierte Alkanolamine mit einer Reinheit von größer 90 Gew.-%, besonders ≥ 97 Gew.-%, insbesondere ≥ 98 Gew.-%, ganz besonders ≥ 99 Gew.-%, eingesetzt werden.

Es können auch Mischungen von Alkanolaminen, wobei dann die oben angegebenen Reinheiten auf jedes Alkanolamin dieser Mischung bezogen sind, oder Lösungen von Alkanolaminen in einem inerten Lösungsmittel, wie z. B. Alkohole (Methanol, Ethanol, iso-Propanol, n-Propanol, n-Butanol, 2-Ethylhexanol), Ether (Tetrahydrofuran, 1,4-Dioxan), Kohlenwasserstoffe (Benzol, Pentan, Petrolether, Toluol, Xylol, Hexan, Heptan, Mihagol) und Wasser, eingesetzt werden.

Die APHA-Farbzahl der eingesetzten Alkanolamine (bezogen auf das nicht säurebehandelte Alkanolamin) beträgt im allgemeinen ≤ 100, insbesondere ≤ 50, beispielsweise ≤ 20.

Bei den im erfindungsgemäßen Verfahren eingesetzten Alkanolaminen handelt es sich um Triethanolamin und N-(2-Aminoethyl)-ethanolamin (AEEA).

Das erfindungsgemäße Verfahren lässt sich wie folgt ausführen:

In einem ersten Verfahrensschritt (Schritt a) wird das in seiner Farbqualität zu verbessernde Alkanolamin oder ein Gemisch der Alkanolamine in flüssiger Phase, optional in Gegenwart eines inerten Lösungsmittels, in einem geeigneten (Rühr)behälter, der mit einem Rückflußkühler ausgerüstet sein kann, mit einer wirksamen Menge von phosphoriger Säure (H₃PO₃), unterphosphoriger Säure (H₃PO₂) oder Verbindungen dieser Säuren, vorteilhaft unter Rühren oder Umpumpen, versetzt und die Mischung über einen Zeitraum von 30 Min. bis 2 Stunden, auf Temperaturen von 150 bis 220°C erwärmt.

Für die bessere Handhabbarkeit kann es dabei von Vorteil sein, die wirksame Menge von phosphoriger Säure, unterphosphoriger Säure oder Verbindungen dieser Säuren in einem geeigneten inerten Verdünnungs- oder Lösungsmittel, wie z. B. Wasser, Alkohole (Methanol, Ethanol, *iso*-Propanol, *n*-Propanol), Ether (Tetrahydrofuran, 1,4-Dioxan) oder einem Alkanolamin (z. B. einem Ethanolamin, wie Monoethanolamin, Diethanolamin, Triethanolamin, N-(2-Aminoethyl)-ethanolamin), wobei hier das Alkanolamin auch dem Alkanolamin-Verfahrensprodukt gleichen kann, in Form einer Lösung oder einer Suspension zuzudosieren.

Die erforderliche Behandlungsdauer des Alkanolamins (oder des Gemischs der Alkanolamine) mit der Phosphorverbindung ergibt sich unter anderem aus dem Grad der Verfärbung des eingesetzten Alkanolamins und dem Ausmaß der gewünschten Entfärbung und/oder Farbstabilität des Alkanolamins. Sie ist in der Regel bei gegebener Temperatur umso größer, je höher der Grad der Verfärbung des im erfindungsgemäßen Verfahren eingesetzten Alkanolamins ist und je höher die Anforderungen an die farbliche Qualität des Verfahrensproduktes sind.

Die Temperatur darf jedoch nicht zu hoch, d. h. in der Regel nicht höher als 250°C, gewählt sein, da ansonsten eine säureinduzierte Degradation des Alkanolamins eintritt, die die Farbqualität des schließlich erhaltenen Alkanolamins negativ beeinflußt.

Die für das jeweilige Alkanolamin günstigsten Temperaturen und Behandlungsdauern sind in einfachen Vorversuchen leicht zu ermitteln.

Bei dieser Behandlung des Alkanolamins mit der Phosphorverbindung ist es von Vorteil, wenn die Mischung während der gesamten Behandlungsdauer oder zeitweise weiter durchmischt (z. B. gerührt oder im Kreislauf umgepumpt) wird.

Weiterhin ist es von Vorteil, wenn die Behandlung des Alkanolamins unter einer Schutzgasatmosphäre (z. B. N₂ oder Ar) durchgeführt wird.

Die Behandlung des Alkanolamins mit der Phosphorverbindung kann auch kontinuierlich in geeigneten Behältern, z. B. in einem Rohrreaktor oder in einer Rührbehälterkaskade, durchgeführt werden.

Die Behandlung des Alkanolamins kann vorteilhaft im Sumpfbehälter einer Destillationskolonne oder in einem Destillationsvorlagegefäß durchgeführt werden.

In einer besonderen Ausgestaltung dieses ersten Verfahrensschritts wird während der Behandlung des Alkanolamins ein Inertgas (z. B. N₂ oder Ar) als Strippstrom durch das Alkanolamin geleitet, um entstehende Leichtsieder, die sich negativ auf die Farbqualität auswirken können, wie z. B. Acetaldehyd oder dessen Folgeprodukte, aus dem Gemisch zu entfernen.

In einer anderen besonderen Ausgestaltung dieses ersten Verfahrensschritts wird das zu behandelnde Alkanolamin im Kreislauf flüssig über einen Wärmetauscher geführt und werden entstehende Leichtsieder, die sich negativ auf die Farbqualität auswirken können, wie z. B. Acetaldehyd, dabei abgeführt.

Bei dem Wärmetauscher kann es sich hierbei um einen offenen Wärmetauscher, wie z. B. einen Fallfilm- oder Wischblattverdampfer, oder einen geschlossenen Wärmetauscher, wie z. B. einen Platten- oder Rohrbündelwärmetauscher, handeln.

Je nach den gewählten Reaktionsbedingungen kann es notwendig sein, die Behandlung des Alkanolamins bei einem Überdruck (z. B. 0,1 bis 50 bar) durchzuführen, um das unerwünschte Entweichen einer oder mehrerer Komponenten aus der Mischung zu vermeiden.

Die phosphorige Säure oder unterphosphorige Säure kann im erfindungsgemäßen Verfahren in monomerer oder auch in polymerer Form, in wasserhaltiger Form (Hydrate) oder als Anlagerungsverbindung (z. B. auf einem anorganischen oder organischen Träger wie SiO₂, Al₂O₃, TiO₂, ZrO₂) eingesetzt werden.

Für das erfindungsgemäße Verfahren sind auch Verbindungen der phosphorigen Säure oder unterphosphorigen Säure, wie Salze (z. B. Dinatriumhydrogenphosphit (Na₂HPO₃), Dinatriumhydrogenhypophosphit (Na₂HPO₂)), Dikaliumhydrogenphosphit (K₂HPO₃), Diammoniumhydrogenphosphit ((NH₄)₂HPO₃)), Amide, Ester (z. B. Triethylphosphit oder Triphenylphosphit) oder deren Anhydride (z. B. P₂O₃) oder Mischungen der vorgenannten Phosphorverbindungen, geeignet.

Dabei können die Salze entweder direkt als solche eingesetzt werden oder durch Mischen, entweder *in situ* oder vor dem Einsetzen in die Behandlung, eines basischen, organischen oder anorganischen Salzes, wie NaOH, KOH, Ca(OH)₂, NH₄OH, Na₂CO₃, K₂CO₃, NaOMe oder NaOEt, gegebenenfalls in einem geeigneten Lösungsmittel, mit der phosphorigen Säure oder unterphosphorigen Säure erhalten werden.

Bevorzugt sind phosphorige Säure (H₃PO₃), unterphosphorige Säure (H₃PO₂), Dinatriumhydrogenphosphit (Na₂HPO₃), Triethylphosphit, Triphenylphosphit und Dinatriumhydrogenhypophosphit (Na₂HPO₂).

Die Menge an den zugesetzten Phosphorverbindungen beträgt 0,02 bis 0,5 Gew.-%, bezogen auf die eingesetzte Menge an Alkanolamin; die Wirkung tritt jedoch auch bei größeren Mengen ein.

Im Falle von sauren zugesetzten Phosphorverbindungen, d. h. solchen mit einem pKₐ-Wert kleiner 4, beträgt die Menge bis 0,5 Gew.-% (bezogen auf die eingesetzte Menge an Alkanolamin), um die Induktion säurekatalysierter Zersetzungsreaktionen des Alkanolamins weitestgehend zu vermeiden.

Im Anschluß an die oben beschriebene Behandlung des Alkanolamins (oder des Gemischs der Alkanolamine) wird das Alkanolamin (oder das Gemisch der Alkanolamine) in einem zweiten Verfahrensschritt (Schritt b) in Gegenwart einer wirksamen Menge einer oder mehrerer der obengenannten Phosphorverbindungen bei vermindertem Druck destilliert oder rektifiziert.

Die Mengen an den Phosphorverbindungen in diesem zweiten Verfahrensschritt liegen im gleichen Bereich wie im ersten Verfahrensschritt.

In einer besonderen Verfahrensausgestaltung erfolgt die Destillation oder Rektifikation des Alkanolamins in Gegenwart der bereits im ersten Verfahrensschritt zugesetzten Phosphorverbindung oder Phosphorverbindungen.

Die Destillation oder Rektifikation des Alkanolamins erfolgt diskontinuierlich oder kontinuierlich bei einem Druck von in der Regel kleiner 100 mbar (100 hPa), beispielsweise bei ca. 10 bis 50 mbar, und bei Sumpftemperaturen von in der Regel 100 bis 250 °C, wobei bei der kontinuierlichen Fahrweise in einer besonderen Ausgestaltung gegebenenfalls vorhandene Leichtsiederanteile über Kopf abgezogen werden und man das Alkanolamin im Seitenabzug erhält.

Der die zugesetzte Phosphorverbindung oder deren Umsetzungsprodukte enthaltende Rückstand der Destillation oder Rektifikation kann in einer besonderen Ausführungsform vollständig oder teilweise in das Verfahren zurückgeführt werden.

Das erfindungsgemäße Verfahren liefert ein in der Farbqualität verbessertes Alkanolamin, das direkt nach seinem Erhalt eine APHA-Farbzahl von 0 bis 30, insbesondere von 0 bis 20, ganz besonders von 0 bis 10, aufweist und das nach einer Säurebehandlung, die wie unten unter 2a) beschrieben innerhalb von 0,5 bis 3 Stunden nach dessen Erhalt ausgeführt wird, eine APHA-Farbzahl von 0 bis 100, insbesondere von 0 bis 60, ganz besonders von 0 bis 40, und einen Absolutwert für die Maßzahl a* nach dem CIE-Lab-System von 0 bis 4, insbesondere von 0 bis 3, ganz besonders von 0 bis 2,5, und einen Absolutwert für die Maßzahl b* nach dem CIE-Lab-System von 0 bis 8, insbesondere von 0 bis 5, ganz besonders von 0 bis 4, aufweist oder das nach einer Säurebehandlung, die wie unten unter 2b) beschrieben innerhalb von 0,5 bis 3 Stunden nach dessen Erhalt ausgeführt wird, eine Gardner-Farbzahl von 0 bis 3, insbesondere von 0 bis 2,5, ganz besonders von 0 bis 2, aufweist.

### Beispiele

### Vorbemerkungen

1a) Temperung und Destillation (Beispiele 1 bis 11)
   Das in den Beispielen 1 bis 11 eingesetzte rohe Triethanolamin (TEA) bestand aus einem technischen Strom der Ethanolaminherstellung, bestehend aus 71 Gew.-% Triethanolamin, 27 Gew.-% Diethanolamin sowie 2 Gew.-% Sonstigem (überwiegend O,N,N-Tris-(2-hydroxyethyl)-ethanolamin, O,N-Bis-(2-hydroxyethyl)-ethanolamin und Bishydroxyethyl-piperazin).
   Eine Probe des in den Beispielen 1 bis 11 eingesetzten rohen TEAs wie oben beschrieben, wies nach einer technischen Destillation eine Reinheit > 99 Gew.-% auf und nach einer Säurebehandlung gemäß 2a) und anschließender Vermessung gemäß 2c) im LICO 200-Gerät sowohl für a* als auch für b* Werte zwischen 4 und 5 auf.
   Die Temperung (= Verfahrensschritt a) wurde durchgeführt, indem das rohe Ethanolamingemisch im Destillationsvorlagegefäß in Gegenwart der in Tabelle 1 aufgeführten Phosphorverbindung bei normalem Druck erhitzt (Temperatur und Dauer siehe Tabelle 1) wurde.
   Die anschließende Destillation des rohen Triethanolamins in Gegenwart der Phosphorverbindung (= Verfahrensschritt b) wurde im 1 1-Maßstab diskontinuierlich bei 0,4 bis 1,0 mbar an einer einstufigen Laborbrücke durchgeführt, wobei die Sumpftemperatur dabei zwischen 160 und 190°C und die Kopftemperatur zwischen 140 und 170°C gehalten wurde.
   Bei der Destillation wurde das Destillat in vier gleichgroßen Fraktionen aufgefangen, von denen die erste typischerweise aus etwa 66 Gew.-% DEA und 33 Gew.-% TEA bestand, die zweite aus 38 Gew.-% DEA und 61 Gew.-% TEA und die dritte und vierte aus jeweils > 97 Gew.-% TEA. Da die ersten beiden Fraktionen kein repräsentatives TEA enthielten, wurden sie verworfen und bei der Farbzahlmessung nicht berücksichtigt.
1b) Temperung und Destillation (Beispiele 12 bis 23)
   Die in Tabelle 2 angegebene Phosphorverbindung wurde unter ca. 12stündigem Rühren und gegebenenfalls leichter Erwärmung (max. 40 bis 60 °C) in reinem AEEA (Reinheit > 99 Gew.-% AEEA; Verunreinigungen: Diethylentriamin (DETA) und Hydroxyethylpiperazin (HEP); Farbqualität: Gardner-Farbzahl nach einer Säurebehandlung wie unter 2b) beschrieben: 6,0, Gardner-Farbzahl nach einer thermischen Behandlung wie unter 2d) beschrieben und anschließender Säurebehandlung wie unter 2b) beschrieben: 6,0) gelöst.
   Das so vorbereitete und unter Stickstoffabdeckung gehaltene Einsatzmaterial wurde durch eine im Ölbad thermostatisierte Rohrschlange (Volumen: 50 ml) gepumpt, wobei Verweilzeit, Menge des Zusatzes und Temperatur wie in Tabelle 2 angegeben variiert wurden (= Verfahrensschritt a).
   Der Austrag der Rohrschlange wurde in einen Wischfilmverdampfer geführt, der bei 50 mbar Druck und einer Öltemperatur zwischen 150 und 170 °C betrieben wurde, so dass ein hinreichend hoher Sumpfablauf (ca. 10 bis 20 % des Volumenstroms) beibehalten wurde, um Ablagerungen an der Heizfläche zu vermeiden. Am Kopf des Wischfilmverdampfers wurde das dampfförmige AEEA abgezogen und in einem Kondensator als Destillat gewonnen (Verfahrensschritt b). Zur verbesserten Trennung wurde ein Stickstoff-Strippstrom von 1 1 N₂/h von unten nach oben durch den Wischfilmverdampfer geleitet.
1c) Temperung und Destillation (Beispiele 24 bis 32)
   Die in Tabelle 3 angegebene Phosphorverbindung (H₃PO₃ oder H₃PO₂) wurde unter ca. 12stündigem Rühren in reinem AEEA (Reinheit > 99 Gew.-% AEEA; Verunreinigungen: Diethylentriamin (DETA) und Hydroxyethylpiperazin (HEP); Farbqualität: Gardner-Farbzahl nach einer Säurebehandlung wie unter 2b) beschrieben: 4,6) gelöst.
   Die weiteren Verfahrensschritte erfolgten wie unter 1 b) beschrieben.
2. Bestimmung der Farbqualität der Alkanolamine
   2a) Säurebehandlung der Alkanolamine (Beispiele 1 bis 11) Zur Amplifizierung der auftretenden Farbeffekte wurden von der zu untersuchenden Alkanolamin-Probe 20 ml mit 1000 ppm Eisessig versetzt und sorgfältig vermischt. Das gut gerührte Gemisch wurde in ein Reagenzglas überführt und 3 h in einem auf 100 °C thermostatisierten Ölbad belassen. Dabei wurde das Gefäß mit einem Stopfen verschlossen und unter Stickstoff gehalten. Der Stopfen war mit einer Kanüle durchstoßen um einen Druckausgleich zu gewährleisten. Nach Ablauf der 3 h wurde das Gefäß in einem Eisbad abgekühlt und direkt anschließend die Farbzahl gemessen (siehe 2c).
   2b) Säurebehandlung der Alkanolamine (Beispiele 12 bis 23 und Beispiele 24 bis 32)
      10 g des Alkanolamins (z. B. Aminoethylethanolamin (AEEA)) wurden in einem 100 ml Erlenmeyerkolben eingewogen und unter Rühren (Magnetrührer) und Kühlung (Eisbad) 12,5 g 32 %ige wässrige Salzsäure innerhalb von einer Minute in drei Portionen eingetragen, anfangs tropfenweise, dann schneller, wobei sich die Lösung erwärmte (max. ca. 30°C). Nach einer Nachkühlzeit von 2 Minuten wurde eine Probe der Lösung in ein Reagenzglas überführt und mit einem mit einer Kanüle durchstoßenen Gummistopfen verschlossen. Das Reagenzglas wurde in einem Wärmebad bei 70 °C für eine Stunde erwärmt. Dann wurde das Reagenzglas in einem Eisbad abgekühlt, der Inhalt direkt im Anschluß in eine Küvette überführt und die Farbzahl gemessen (siehe 2c).
      [Eine Säurebehandlung eines Alkanolamins zur Verstärkung von Farbeffekten wurde allgemein in JP-A-62 019 558 (Derwent Abstract Nr. 87-067647/10) und JP-A-62 005 939 (Derwent Abstract Nr. 87-047397/07) beschrieben, wonach TEA mit Essigsäure, Zitronensäure, Schwefelsäure, Salzsäure oder Phosphorsäure behandelt (neutralisiert) wird.]
   2c) Farbzahlmessung (Beispiele 1 bis 32)
      Generell wurde die Farbzahl der zuvor säurebehandelten Probe max. 3 h nach Abkühlen gemessen, um eine Nachfärbung nach der Säurebehandlung (infolge von Alterung) möglichst gering zu halten.
      In einer spektralen Farbmessung wurden die Werte für die Maßzahlen a* und b* nach dem CIE-Lab-System (nach Judd und Hunter (CIE = Comission International d'Eclairage, Paris); (vergl. DIN 6174)), der APHA-Wert (entsprechend DIN-ISO 6271) und die Gardner-Farbzahl (DIN ISO 4630) bestimmt.
      Die Bestimmung der a*-, b*-, und APHA-Werte (APHA- = Hazen- = Pt/Co-Farbzahl) in den Beispielen 1 bis 11 erfolgte standardmäßig in einem LICO 200-Gerät der Firma Dr. Lange in einer 5 cm (Schichtdicke) Küvette (Volumen ≈ 10 ml).
      Die Bestimmung der Gardner- und APHA-Werte in den Beispielen 12 bis 32 erfolgte standardmäßig in einem Liquid Tester LTM1 der Firma Dr. Lange in einer 11 mm (Innendurchmesser) Rundküvette.
      Der a*-Wert gibt die Rot-/Grünfärbung der Probe an (ein positiver a*-Wert gibt den roten Farbanteil an, ein negativer a*-Wert den grünen Farbanteil) und der b*-Wert den Gelb-/Blauanteil (ein positiver b*-Wert gibt den gelben Farbanteil an, ein negativer b*-Wert den blauen Farbanteil). Anzustreben ist besonders ein geringerer absoluter a*-Wert als im Ausgangsmaterial vor der Durchführung des erfindungsgemäßen Verfahrens.
      Die in den Tabellen Nr. 1, 2 und 3 angegebenen a*-, b*-, Gardner- und APHA-Werte beziehen sich stets auf die Proben nach der entsprechend durchgeführten Säurebehandlung.
   2d) Lagerversuche (Beispiele 12 bis 23)
      Um die Farbstabilität von AEEA zu untersuchen, wurden 50 g des nach der Destillation erhaltenen AEEAs in einem Dreihalskolben mit aufgesetztem Wasserkühler unter Stickstoff für 3 Stunden auf 90 °C Innentemperatur erwärmt. Nach Abkühlen auf Raumtemperatur wurde wie oben unter 2b) beschrieben säurebehandelt und wie oben unter 2c) beschrieben die Farbzahl gemessen. Durch diese thermische Behandlung des AEEAs bei 90°C für 3 h wurde eine Lagerung über ca. 40 Tage bei 20°C simuliert.

### Ergebnisse

Die Ergebnisse der Beispiele Nr. 1 bis 11, 12 bis 23 und 24 bis 32 sind in den Tabellen 1, 2 und 3 dargestellt.

Bei den Beispielen 1 bis 4, 8, 12, 13, 15, 18, 21 bis 27, 29, 30 und 32 handelt es sich um Vergleichsbeispiele.

Beispiel 1 stellt die Destillation des TEA-Rohgemisches ohne vorherige Temperung als Vergleichswert dar, Beispiel 2 dagegen mit Temperung (60 Min., 180°C), jedoch jeweils ohne erfindungsgemäßen Zusatz der Phophorverbindung.

In beiden Fällen ist der a*- bzw. b*-Absolutwert des Produkts unakzeptabel hoch.

Durch Zusatz von H₃PO₃ oder Na₂HPO₃ während der Destillation, jedoch ohne vorherige Temperung, wurde die Farbqualität nicht ausreichend verbessert (Beispiele 3 und 4): die a*- und/oder b*-Absolutwerte blieben unakzeptabel hoch.

### Beispiele 5 bis 11:

Durch vorherige thermische Behandlung (Temperung) des Alkanolamins in Gegenwart der beschriebenen Phosphorverbindungen kann eine Verbesserung der Farbqualität auf die geforderten Farbzahlen und a*- und b*-Werte erreicht werden. Dabei ist H₃PO₃ effektiver als Na₂HPO₃ was die Absenkung sowohl des a*- als auch des b*-Absolutwertes betrifft. Na₂HPO₃ ist wirksamer für die Absenkung allein des a*-Absolutwertes, läßt jedoch den b*-Wert weniger beeinflußt.

### Beispiele 13 bis 23:

Durch die erfindungsgemäße Behandlung des AEEAs mit H₃PO₃ wurde in jedem Fall die Farbqualität deutlich verbessert (Vergleich mit Beispiel 12).

Die Farbzahl des Verfahrensproduktes war umso günstiger, je länger das AEEA in Gegenwart von H₃PO₃ vorbehandelt wurde (Beispiel 14 vs. 13, 17 vs. 16 vs. 15 sowie 20 vs. 19 vs. 18).

Außerdem ist bei zunehmenden Temperaturen im ersten Verfahrensschritt ein positiver Effekt festzustellen (Beispiel 19 vs. 14).

Weiterhin wirkt sich die zugesetzte Menge an H₃PO₃ positiv auf die Farbqualität aus (Beispiel 22 vs. 19 vs. 16).

Wird AEEA in Gegenwart zu großer Mengen an H₃PO₃ bei zu hoher Temperatur zu lange getempert (= Verfahrensschritt a) (Beispiel 23 vs. 20), so wird eine Verschlechterung der Farbzahl beobachtet (säureinduzierte Degradation).

### Beispiele 24 bis 32:

Beispiel 24 zeigt als Vergleich das Ergebnis bezüglich der Farbzahl einer einfachen Destillation des AEEAs über einen Sambay-Verdampfer.

Gemäß der Beispiele 24, 27 und 30 führt eine Destillation des AEEAs in Gegenwart der Phosphorverbindung ohne vorherige erfindungsgemäße Temperung (Schritt a) zu keiner Verbesserung der Farbzahl des Alkanolamins.

Die Beispiele 28 und 31 sind erfindungsgemäß und zeigen die Verbesserung der Farbqualität.

**Tabelle 1**

| | | Temperung (Verfahrensschritt a) | | Fraktion 3 (der Destillation) | | | Fraktion 4 (der Destillation) | | |
|---|---|---|---|---|---|---|---|---|---|
| Beispiel Nr. | Zusatz | Dauer [Min.] | Temperatur [°C] | a* | b* | APHA | a* | b* | APHA |
| 1 | - | - | - | -9,3 | 65,6 | 530 | -6,1 | 24,6 | 168 |
| 2 | - | 60 | 180 | -10,7 | 71,2 | 567 | -8,8 | 30,5 | 254 |
| 3 | 2500 ppm H₃PO₃ | - | - | 4,3 | 3,0 | 35 | 5,1 | 3,3 | 39 |
| 4 | 2200 ppm Na₂HPO₃ | - | - | 2,4 | 16,3 | 130 | 3,3 | 14,1. | 118 |
| 5 | 2500 ppm H₃PO₃ | 30 | 180 | 1,7 | 1,4 | 15 | 2,0 | 2,2 | 22 |
| 6 | 2500 ppm H₃PO₃ | 60 | 180 | 1,3 | 1,2 | 13 | 1,7 | 1,6 | 17 |
| 7 | 4000 ppm H₃PO₃ | 60 | 180 | 1,0 | 0,9 | 10 | 1,4 | 1,1 | 12 |
| 8 | 4000 ppm H₃PO₃ | 10 | 180 | 2,2 | 2,7 | 26 | 2,1 | 3,0 | 28 |
| 9 | 4000 ppm H₃PO₃ | 60 | 150 | 2,0 | 2,6 | 25 | 2,2 | 2,9 | 28 |
| 10 | 2200 ppm Na₂HPO₃ | 60 | 180 | 0,9 | 6,8 | 54 | 0,4 | 7,3 | 56 |
| 11 | 2000 ppm P(OPh)₃ | 60 | 180 | 1,7 | 3,2 | 29 | 2,1 | 4,6 | 41 |

**Tabelle 2**

| Beispiel Nr. | Zusatz | Rohrschlange | | Sambay-Destillation | | | | Nach Lagerung | |
|---|---|---|---|---|---|---|---|---|---|
| | | Temp. (°C) | VWZ (Min.) | Temp. (°C) | Druck (mbar) | Farbzahl (nach Säurebehandlung) APHA Gardner | | Farbzahl (nach Säurebehandlung) APHA Gardner | |
| 12 | --- | 180 | 30 | 160 | 50 | 714 | 3,7 | 762 | 3,9 |
| 13 | 4000 ppm H₃PO₃ | 150 | 10 | 170 | 50 | 430 | 2,5 | 502 | 2,8 |
| 14 | 4000 ppm H₃PO₃ | 150 | 30 | 160 | 50 | 165 | 1,1 | 312 | 1,9 |
| 15 | 2000 ppm H₃PO₃ | 180 | 10 | 170 | 50 | 198 | 1,2 | 252 | 1,6 |
| 16 | 2000 ppm H₃PO₃ | 180 | 30 | 160 | 50 | 145 | 0,9 | 177 | 1,1 |
| 17 | 2000 ppm H₃PO₃ | 180 | 60 | 153 | 50 | 35 | 0,2 | 75 | 0.,4 |
| 18 | 4000 ppm H₃PO₃ | 180 | 10 | 170 | 50 | 100 | 0,7 | 125 | 0,8 |
| 19 | 4000 ppm H₃PO₃ | 180 | 30 | 160 | 50 | 100 | 0,6 | 110 | 0,7 |
| 20 | 4000 ppm H3PO₃ | 180 | 60 | 153 | 50 | 25 | 0,1 | 46 | 0,3 |
| 21 | 10000 ppm H₃PO₃ | 180 | 10 | 170 | 50 | 36 | 0,2 | 40 | 0,3 |
| 22 | 10000 ppm H₃PO₃ | 180 | 30 | 160 | 50 | 14 | 0,2 | 48 | 0,3 |
| 23 | 10000 ppm H₃PO₃ | 180 | 60 | 153 | 50 | 70 | 0,4 | 101 | 0,6 |

| | | | | | | | | | |
|---|---|---|---|---|---|---|---|---|---|
| (VWZ = Verweilzeit) | | | | | | | | | |

**Tabelle 3**

| Beispiel Nr. | Zusatz. | Rohrschlange (Schritt a) | | Farbzahlen nach Sambay-Destillation und Säurebehandlung (HCl) | |
|---|---|---|---|---|---|
| | | Temp. (°C) | VWZ (Min.) | APHA | Gardner |
| 24 | --- | RT | 30 | 505 | 2,9 |
| 25 | --- | 180 | 30 | 578 | 3,3 |
| 26 | --- | 180 | 10 | 541 | 3,1 |
| 27 | 4000 ppm H₃PO₃ | RT | 30 | 515 | 2,9 |
| 28 | 4000 ppm H3PO₃ | 180 | 30 | 131 | 0,5 |
| 29 | 4000 ppm H₃PO₃ | 180 | 10 | 149 | 0,6 |
| 30 | 4000 ppm H₃PO₂*) | RT | 30 | 521 | 3,0 |
| 31 | 4000 ppm H₃PO₂*) | 180 | 30 | 216 | 1,0 |
| 32 | 4000 ppm H₃PO₂*) | 180 | 10 | 464 | 2,6 |

| | | | | | |
|---|---|---|---|---|---|
| (VWZ = Verweilzeit) (RT = Raumtemperatur = 22 °C) *) als 50 %ige wässrige Lösung. | | | | | |

## Patentansprüche

1. Verfahren zur Herstellung von Triethanolamin oder N-(2-Aminoethyl)-ethanolamin mit verbesserter Farbqualität, **dadurch gekennzeichnet, dass** man das Alkanolamin, dessen Reinheit größer 70 Gew.-% beträgt, mit einer wirksamen Menge von phosphoriger oder unterphosphoriger Säure oder deren Verbindungen zunächst bei Temperaturen von 150 bis 220°C über einen Zeitraum von 30 Min. bis 2 Stunden behandelt (Schritt a) und anschließend in Gegenwart einer wirksamen Menge einer dieser Phosphorverbindungen destilliert (Schritt b), wobei man die Behandlung des Alkanolamins in Schritt a und die Destillation des Alkanolamins (Schritt b) jeweils in Gegenwart von 0,02 bis 0,5 Gew.-% der Phosphorverbindung, bezogen auf die eingesetzte Menge an Alkanolamin, durchführt.

2. Verfahren nach Anspruch 1, **dadurch gekennzeichnet, dass** es sich bei den Phosphorverbindungen um Dinatriumhydrogenphosphit (Na₂HPO₃) Triethylphosphit, Triphenylphosphit oder Dinatriumhydrogenhypophosphit (Na₂HPO₂) handel.

3. Verfahren nach einem der Ansprüche 1 bis 2, **dadurch gekennzeichnet, dass** das hergestellte Alkanolamin nach einer dreistündigen Behandlung mit 1000 ppm Eisessig bei 100°C eine APHA-Farbzahl (DIN ISO 6271) von 0 bis 100, einen Absolutwert für die Maßzahl a* nach dem CIE-Lab-System von 0 bis 4 und einen Absolutwert für die Maßzahl b* nach dem ClE-Lab-System von 0 bis 8 aufweist oder nach einer einstündigen Behandlung mit der 1,25 fachen Gewichtsmenge 32 %iger wässriger Salzsäure bei 70°C eine Gardner-Farbzahl (DIN ISO 4630) von 0 bis 3 aufweist.

## Claims

1. A process for the preparation of triethanolamine or N-(2-aminoethyl)ethanolamine having improved color quality, which comprises treating the alkanolamine, the purity of which is greater than 70% by weight, with an effective amount of phosphorous acid or hypophosphorous acid or compounds thereof initially at temperatures of from 150 to 220°C over a period of 30 min to 2 hours (step a), and then distilling it in the presence of an effective amount of one of these phosphorus compounds (step b) where the treatment of the alkanolamine in step a and the distillation of the alkanolamine (step b) are in each case carried out in the presence of from 0.02 to 0.5% by weight of the phosphorus compound, based on the amount of alkanolamine used.

2. The process according to claim 1, wherein the phosphorus compounds are disodium hydrogenphosphite (Na₂HPO₃) , triethyl phosphite, triphenyl phosphite or disodium hydrogenhypophosphite (Na₂HPO₂).

3. The process according to any one of claims 1 to 2, wherein the prepared alkanolamine, following treatment for three hours with 1000 ppm of glacial acetic acid at 100°C, has an APHA color number (DIN ISO 6271) of from 0 to 100, an absolute value for the numerical measure a* according to the CIE Lab system of from 0 to 4 and an absolute value for the numerical measure b* according to the CIE Lab system of from 0 to 8, or following treatment for one hour with 1.25 times the amount by weight of 32% strength aqueous hydrochloric acid at 70°C, a Gardner color number (DIN ISO 4630) of from 0 to 3.

## Revendications

1. Procédé pour la préparation de triéthanolamine ou de N-(2-aminoéthyl)-éthanolamine à qualité de couleur améliorée, **caractérisé en ce que** d'abord on traite l'alcanolamine, dont le degré de pureté est supérieur à 70 % en poids, par une quantité efficace d'acide phosphoreux ou hypophosphoreux ou leurs dérivés, pendant une durée de 30 minutes à 2 heures à des températures de 150 à 220°C (étape a) et ensuite on la distille en présence d'une quantité efficace d'un de ces composés phosphorés (étape b), en effectuant le traitement de l'alcanolamine dans l'étape a et la distillation de l'alcanolamine (étape b) chaque fois en présence de 0,02 à 0,5 % en poids du composé phosphoré, par rapport à la quantité utilisée d'alcanolamine.

2. Procédé selon la revendication 1, **caractérisé en ce que** les composés phosphorés consistent en monohydrogénophosphite de sodium (Na₂HPO₃), phosphite de triéthyle, phosphite de triphényle ou monohydrogénohypophosphite de sodium (Na₂HPO₂).

3. Procédé selon la revendication 1 ou 2, **caractérisé en ce que** l'alcanolamine obtenue après un traitement de trois heures par 1 000 ppm d'acide acétique glacial à 100°C présente un indice colorimétrique APHA (DIN ISO 6271) de 0 à 100, une valeur absolue de la coordonnée chromatique a* selon le système CIE-Lab de 0 à 4 et une valeur absolue de la coordonnée chromatique b* selon le système CIE-Lab de 0 à 8 ou, après un traitement d'une heure par la quantité en poids x1,25 d'acide chlorhydrique aqueux à 32%, à 70°C, un indice colorimétrique de Gardner (DIN ISO 4630) de 0 à 3.
